(19) Europäisches Patentamt
European Patent Office
Office européen des brevets

(11) Publication number: **0 103 919**
**B1**

# (12) EUROPEAN PATENT SPECIFICATION

(45) Date of publication of patent specification: **30.12.86**

(51) Int. Cl.$^4$: **C 07 C 127/19**

(21) Application number: **83201184.5**

(22) Date of filing: **12.08.83**

## (54) Process for producing substituted asymmetric ureas.

(30) Priority: **25.08.82 IT 2296782**

(43) Date of publication of application:
**28.03.84 Bulletin 84/13**

(45) Publication of the grant of the patent:
**30.12.86 Bulletin 86/52**

(84) Designated Contracting States:
**AT BE CH DE FR GB LI LU NL SE**

(56) References cited:
**GB-A-1 372 537**
**US-A-3 161 676**

(73) Proprietor: **ANIC S.p.A.**
**Via Ruggero Settimo, 55**
**I-90139 Palermo (IT)**

(72) Inventor: **Romano, Ugo**
**Via 25 Aprile 10**
**I-20059 Vimercate Milan (IT)**
Inventor: **Fornasari, Giancarlo**
**Via Valignani 13**
**I-65100 Pescara (IT)**
Inventor: **Sgambato, Umberto**
**Via Martinengo 30**
**I-20139 Milan (IT)**

(74) Representative: **Roggero, Sergio et al**
**Ing. Barzanò & Zanardo Milano S.p.A. Via Borgonuovo 10**
**I-20121 Milano (IT)**

**The file contains technical information submitted after the application was filed and not included in this specification**

**EP 0 103 919 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for preparing substituted asymmetric ureas, useful as active ingredients in pesticides and weed-killers.

Conventionally, asymmetric ureas are prepared by reacting an aromatic isocyanate, or a carbamoyl chloride, with an aliphatic amine, such as disclosed in "Pesticides Manual Encyclopedia," by M. Sitting, 1979 Edn.: this approach is objectionable under many respects, the most prominent of which is toxicity.

Toxic chemicals, such as carbamoyl chlorides and isocyanates, require quite special precautions in shipping, storage and handling: moreover, the processes for producing such starting materials, as isocyanates and carbamoyl chlorides, involve toxicity problems, especially due to the use of phosgene.

All these compounds moreover, originate environmental pollution problems when effluents are to be discharged.

Attempts to prepare asymmetric ureas by overcoming these drawbacks have been the processes starting from aliphatic amines and carbamates, aided by the use of catalyst: typical is the EP—A—82200632.6 filed May 24,1982.

A catalytic process starting from dialkyl carbamates and aromatic amines is known from the US Patents 4,268,683 and 4,268,684.

The UK Patent GB 1 372 537 (Halcon) prepares N-substituted carbamates by reacting an organic carbamate with a primary, or a secondary, amine and a Lewis' acid is used as the catalyst, and the US Patent 3 161 767 (Adams) teaches how to prepare alkyl ureas by reacting a primary, or a secondary, amine with a carbamic acid ester, and the catalyst is, again, a Lewis' acid.

Now, therefore, in order to obviate to the above enumerated shortcomings, the invention provides a simplified, 2-stage catalytic process for preparing asymmetric ureas,

According to the invention, therefore, there is provided a catalytic process for preparing substituted asymmetric ureas having the general formula:

$$HR_1NCONR_2R_3$$

wherein $R_1$ is an aryl radical optionally substituted in its ring;

$R_2$ is an alkyl radical having from 1 to 10 carbon atoms,

or a cycloalkyl radical having 5 or 6 carbon atoms, and

$R_3$ is a hydrogen atom or $R_2$,

by a 2-stage run, in the first stage of which a dialkl carbonate having the formula RO—CO—OR, wherein R is an alkyl radical having from 1 to 4 carbon atoms, is reacted with an aromatic amine having the formula $R_1NH_2$, and the unreacted dialkyl carbonate and the concurrently produced alcohol are distilled off as the reaction proceeds, whereas in the second stage the residual products of the first stage are reacted with an aliphatic amine having the formula $R_2R_3NH$, characterized in that in the first stage the molar ratio of the dialkyl carbonate to the aromatic amine is comprised between 1:1 and 5:1, and from 50% to 95% of the aromatic amine concerned is converted, and in the second stage the as-produced intermediate carbamate coming from the first stage is reacted with the aliphatic amine in the presence of the residual unreacted aromatic amine.

Examples of dialkyl carbonates usable for the process of the invention are dimethyl carbonate, diethylcarbonate, dipropylcarbonate, diisopropylcarbonate and dibutylcarbonate.

Examples of amines $R_1NH_2$ which can be used for the process of this invention are aniline, 3,4 - bichloroaniline, 3-chloro-4-methyl aniline, 3 - chloroaniline, 4 - chloroaniline and 2 - trifluoromethylaniline.

Examples of amines $R_2R_3NH$ which can be used to the intended purpose are: dimethylamine, diethylamine, di-nor. propylamine, di-nor. butylamine, nor.butylmethylamine, cyclohexylamine, methylamine, ethylamine, propylamine and butylamine.

The catalysts which are useful for the purpose intended herein are members selected from the group consisting of the Lewis' acids, the alkoxides or the hydroxides of alkali metal, alkaline earth metals, or of zinc. Examples of these are: sodium methoxide, sodium ethoxide, lithium methoxide, lithium ethoxide, zinc methoxide, zinc ethoxide, zinc acetate, zinc chloride and tin chloride. The first reaction stage is carried out by contacting the dialkylcarbonate concerned with the aromatic amine, in a molar ratio of the one to the other equal to, or higher than 1:1, at a temperature of from 70° to 160°, in the presence of an amount of catalyst which is from 1 to 10 mol per 100 mols of the aromatic amine, until the reactants are but partially converted. It has been ascertained, in practice, that final reaction products having the expected purity are obtained if the conversion of the aromatic amine is stopped to a value not exceeding 95%. The lower limit of the conversion of the aromatic amine has no criticality but, for economical reason, it is preferred not to fall below about 50%.

The working conditions which are preferred for the first reaction stage are as follows: molar ratio of the dialkylcarbonate to the aromatic amine over 1:1 up to 5:1, temperature from 120°C to 160°C, catalyst from 5 to 10 mol per 100 mol of amine in the case of using, as catalysts, alkoxides, oxides or hydroxides, and from 1 to 5 mol of catalyst per 100 mol of amine when using the Lewis' acids as the catalysts, the amine conversion rating being from 50% to 90%. The times which are required to bring about these conversions generally vary from 1 to 6 hours. From the reaction mixture as obtained in the first reaction stage, the unreacted dialkylcarbonate and the co-produced alcohol are removed by distilling them off under an atmospheric pressure or a reduced pressure. The unreacted aromatic

amine is not removed at this stage of the reaction, since it is more favorable to effect its separation from the mixture which is discharged on completion of the second reaction stage.

It has been ascertained, in fact, that in said second stage the reaction runs in a quick and selective way in the presence of the aromatic amine.

More particularly, during progress of said second stage, the distillation residue comes into contact with the aliphatic amine with a molar ratio of the aliphatic amine to the dialkylcarbonate which had been interacted in the first stage equal to or higher than 1:1, at a temperature of from 70°C to 160°C, in the presence of the catalyst charged in the first stage, the corresponding substituted asymmetric urea being thus obtained.

The conditions which are preferred for the second reaction stage are as follows: molar ratio of the aliphatic amine to the dialkylcarbonate which had been interacted in the first stage higher then 1:1 up to 5:1, and temperatures from 120°C to 140°C. The times which are required for completing the reaction in the second stage vary from 1 to 3 hours as a rule.

In both stages, it is also possible to work within a solvent, such as toluene, zylene, hexane, dodecylbenzene, decahydronaphthalene, tetrahydronaphthalene, diethyl ether and methyltert.butyl ether.

The substituted asymmetric urea thus obtained can be withdrawn from the reaction mixture with conventional procedures such as precipitation or by distilling off the unreacted reactants, the co-produced alcohol and the solvent which may have been added to the system. The substituted asymmetric urea thus prepared can finally be subjected to purification, for example by crystallization from a solvent.

The experimental examples reported hereinafter are illustrative and do not limit the scope of the invention.

Example 1

A stainless-steel autoclave is used, having a volume of 150 mls, and equipped with a jacket and a thermocouple, a magnetic stirrer and a thermostatically controlled fractionation column connected to a condenser. The autoclave is charged with 7.76 g (0.083 mol) of aniline, 11.8 g (0.10 mol) of DEC (diethylcarbonate) and 0.68 g (0.01 mol) of sodium ethoxide and the reaction is carried out during 5 hours at 120°C. The pressure within the autoclave attains 2 $Kg/cm^2$. At the end of the period of time aforesaid, the residual DEC and the ethanol (by-product) are distilled off under a pressure lower than the ambient pressure.

The residue of the distillation is supplemented with 50 g of toluene and 13 g (0.10 mol) of di - norbutylamine. The reaction is now carried out for 3 hours at 120°C and ethanol is distilled off as it is being formed. At the end of the period of time aforementioned the unreacted toluene, dibutylamine and aniline are distilled off, under a pressure which is lower than the ambient pressure and the residue is recovered.

A conversion of aniline is determined, which is 62.5 molar % with a yield of useful reaction product, which is 1-dibutyl-3-phenylurea, equal to 60.3% (assessed after recrystallization from nor.hexane) whereas N - ethylethylcarbanylate is obtained with a yield of 2%.

Example 2

The reactor of Example 1 is charged with 7.76 g (0.083 mol) of aniline, 11.8 g (0.10 mol) of DEC and 0.068 g of sodium ethoxide and the reaction is caused to proceed during 5 hours at 120°C. At the end of this period of time, the unreacted DEC and the ethanol which has been formed are distilled off under a pressure lower than the ambient pressure and to the residue there are added 60 g of toluene and 8.3 g (0.085 mol) of cyclohexylamine. The reaction is carried out during 2.5 hours at 120°C while ethanol is distilled off as it is being formed. At the end of the period of time in question, toluene, cyclohexylamine and aniline which did not react are distilled off working under a pressure lower than the ambient pressure, and the residue is recovered. A conversion of aniline is determined, which is 63.5 molar %, with a yield of the useful reaction product, which is 1 - cyclohexyl - 3 - phenylurea, equal to 58.9% (assessed upon recrystallization), whereas the yield of N - ethyl - ethylcarbanylate is 1.9%.

Example 3

The reactor of Example 1 is charged with 12.20 g (0.086 mol) of 3 - chloro - 4 - methylaniline, 12.25 g (0.10 mol) of DEC and 0.7 g (0.01 mol) sodium ethoxide. The reaction is carried out for 5 hours at 120°C and at the end of this period of time the unreacted DEC and the by-product, enthanol, are distilled off working under a pressure which is lower than the ambient pressure. To the residue there are added 10.4 g (0.10 mol) of di-nor.propylamine and 50 g of toluene. The reaction is carried out again for 3 hours at 115°C and ethanol is distilled off as it is being produced. On completion of the reaction there is determined a conversion of the 3 - chloro - 4 - methylanine which is equal to 64 molar %, with a yield of the useful product, 1 - (3 - chloro - 4 - methylphenyl) - 3 - dipropylurea equal to 61.1% and with a yield of 2.1% for the N - ethyl - ethyl - carbanylate of the 3 - chloro - 4 - methylaniline.

Example 4

A 500-ml stainless stell autoclave is used, which is equipped with magnetic stirrer, heating jacket, thermocouple and thermostatically controlled fractionation column which is connected to a condenser. The autoclave is charged with 64.8 g (0.40 mol) of 3,4 - dichloroaniline, 56.64 g (0.48 mol) of DEC and 15.1 mls of a 18% solution of sodium ethoxide (the solution is in terms of weight/volume and the quantity used

corresponds to 0.04 mol of sodium ethoxide). The reaction is carried out for 5 hours at 120°C and in the reactor the pressure attains a maximum value of 2 $Kg/cm^2$. At the end of that period of time, the residual DEC and the as-formed ethanol are distilled off under a pressure lower than the ambient pressure. To the residue there are added 40 mls of decahydronaphthalene and 44.7 g (0.99 mol) of dimethylamine. The reaction is allowed to proceed during 3 hours at 120°C, and a maximum pressure of about 5 $Kg/cm^2$ is attained within the reactor. At the end of this period of time, the unreacted dimethylamine is vented off the reactor ceiling and the reaction mixture is transferred into a filtration device wherein the solids are separated from the solvent and from the unreacted amine.

The recovered solid is white and crystalline and has a melting point of 152°C—153°C. The IR spectra, mass spectra and NMR spectra confirm that the compound is 3 - (3,4 - bichlorophenyl) - 1,1 - dimethylurea. In addition, a conversion of dichloroaniline is detected, which is equal to 53.1 molar %, with a yield of 50.1% of the useful reaction product (assessed upon recrystallization) and with a yield of 1.5% of N - ethyl - ethylcarbanylate of the 3,4 - dichloroaniline.

Example 5

The reactor of Example 1 is charged with 17.4 g (0.11 mol) of 3,4 - dichloroaniline, 50 g (0.55 mol) of DMC (dimethylcarbonate) and 0.64 g (0.005 mol) of zinc dimethoxide and the reaction is carried out with stirring for 6 hours at 140°C, a maximum pressure being attained, which is about 10 $Kg/cm^2$. At the end of said period of time, the unreacted DMC and the as-formed methanol are distilled off working under a pressure which is slightly below the ambient pressure. To the residue of the distillation there are added 40 mls of xylene and 14 g (0.31 mol) of dimethylamine and the reaction is carried out for 3 hours at 120°C.

At the end of said period of time the unreacted dimethylamine is evaporated off and the reaction mixture is filtered. The solid which is thus separated is dried and is identified by analysis as 3 - (3,4 - bichlorophenyl - 1,1 - dimethylurea.

The GLC-analysis (gas-liquid chromatograhy) of the liquid filtrate has permitted to determine a conversion of 3,4 - dichloroaniline as high as 63 molar %, with a yield of the useful product of the reaction equal to 55% (evaluated upon recrystallization. As a by-product of the reaction, N - methyl - 3,4 - dichloroaniline is obtained with a yield of 2%.

Example 6

The reactor f Example 1 is charged with 32.4 g (0.20 mol) of 3,4-dichloroaniline, 90 g (1.0 mol) of DMC and 1.90 g (0.01 mol) of zinc acetate. The reaction is conducted at 140°C for 6 hours and a maximum pressure of 9 $Kg/cm^2$ is attained. At the end of this period of time the unreacted DMC and the as-formed methanol are distilled off while working under a pressure slightly below the ambient pressure. To the residue there are added 32.9 g (0.73 mol) of dimethylamine and the reaction is carried out at 120°C for 3 hours, a maximum pressure of about 11 $Kg/cm^2$ being attained. At the end of that period of time, the unreacted dimethlamine is evaporated off and the reaction mixture is filtered. A crystalline solid is recovered, which melts at 151°C—152°C and is analytically identified as 3 - (3,4 - bichlorophenyl) - 1,1 - dimethylurea. A conversion of the dichloroaniline is experienced, which is equal to 80 molar % with a yield of 76% of the useful reaction product (evaluated upon recrystallization) and with a yield of 0.5% of N - methyldichloroaniline.

Example 7

The reactor of Example 1 is charged with 32.4 g (0.20 mol) of 3,4 - bichloroaniline, 21.6 g (0.24 mol) of DMC and 1.9 g (0.015 mol) of zinc carbonate. The reaction is conducted for 6 hours at 140°C and, at the end of this period of time, the unreacted DMC and the as-formed methanol are distilled off, working under a pressure which is slightly below the ambient pressure. To the residue there are added 35 mls of toluene and 28.8 g (0.64 mol) of dimethylamine and the reaction is carried out for 3 hours at 120°C, a maximum pressure of about 12 $Kg/cm^2$ being attained. At the end of that period of time, the reaction mixture is filtered and there are recovered 22.35 g of a solid product which melts at 150°C—152°C and which is identified analytically as being 3 - (3,4 - bichlorophenyl) - 1 - dimethylurea. A conversion of 3,4 - bichloroaniline is experienced, which is as high as 54 molar %, with a yield of 48.2% of the useful reaction product, evaluated after recrystallization and with a yield of 0.5% of N - methyl - 3,4 - bichloroaniline.

Example 8

The reactor of Example 1 is charged with 9.88 g (0.1062 mol) of aniline, 50 g (0.5556 mol) of DMC and 0.92 g (0.005 mol) of zinc acetate. The reaction is carried out with stirring and under a nitrogen blanket at 140°C for 6 hours. At the end of that period of time, the reactor is fed with 10 mls of didecylbenzene, whereafter both the residual DMC and the newly formed methanol are distilled off, working under a pressure which is slightly below the ambient pressure. To the residue there are added 15.3 g (0.33 mol) of dimethylamine and the reaction is carried out for 3 hours at 120°C. At the end of such period of time, the unreacted dimethylamine is evaporated off and the reaction mixture is filtered. The solid collected on the filter is washed with 20 mls of ethyl ether and dried. There are thus recovered 13.28 g (0.081 mol) of a solid product, which melts at 131°C—133°C and is identified by NMR and MS analyses as 3 - phenyl - 1,1 - dimethylurea. A conversion of aniline is experienced which is as high as 78 molar %, with a yield of the useful

reaction product as high as 76.2% and a yield of 0.6% of N-methylaniline.

Example 9

The reactor of Example 1 is charged with 32.4 g (0.20 mol) of 3,4 - dichloroaniline, 90.0 g (1.0 mol) of DMC and 1.9 g (0.01 mol) of zinc acetate. The reaction is carried out with stirring and under a nitrogen blanket for 6 hours at 140°C. At the end of that period of time, the unreacted DMC and the as-formed methanol are distilled off under a pressure slightly below the atmospheric pressure. To the residue there are added 30 mls of toluene and 55.8 g (0.64 mol) of N - butylmethylamine and the reaction is carried out for 3 hours at 120°C. At the end of that period of time the unreacted N - butylmethylamine is distilled off and the reaction mixture is filtered. A white crystalline solid is recovered, which melts at 99°C—101°C and is identified as N - butyl - N' - (3,4 - dichlorophenyl) - N - methylurea. A conversion of the 3,4 - dichloroaniline as high as 78 molar % is experienced, with a yield of 74% of the useful reaction product and with a yield of 1% in terms of N - methyldichloroaniline.

Example 10

The reactor of Example 4 is charged with 64.8 g (0.40 mol) of 3,4 - dichloroaniline, 72.1 g (0.80 mol) of DMC and 3.8 g (0.02 mol) of zinc acetate. The reaction is carried out at 140°C for 6 hours. At the end of that period of time the unreacted DMC and the newly formed methanol are distilled off under a pressure below the ambient pressure. To the residue there are added 62.3 g (1.38 mol) of dimethylamine and 60 mls of zylene and the reaction is conducted for 3 hours at 120°C. At the end of that period the unreacted dimethylamine is evaporated off and the residue is filtered. The solid collected on the filter is washed with nor.hexane and there are recovered 63.2 g of a product which has been identified as 3 - (3,4 - dichlorophenyl) - 1,1 - dimethylurea.

A conversion of 3,4 - dichloroaniline as high as 71.5 molar % is experienced, with a yield of 68.1% of the useful reaction product (evaluated upon recrystallization) and with a yield of 1% in terms of N - methyl - 3,4 - dichloroaniline.

**Claims**

1. A catalytic process for preparing substituted asymmetric ureas having the general formula:

$$HR_1NCONR_2R_3$$

wherein $R_1$ is an aryl radical optionally substituted in its ring;

$R_2$ is an alkyl radical having from 1 to 10 carbon atoms,

or a cycloalkyl radical having 5 or 6 carbon atoms, and

$R_3$ is a hydrogen atom or $R_2$,

by a 2-stage run, in the first stage of which a dialkyl carbonate having the formula RO—CO—OR, wherein R is an alkyl radical having from 1 to 4 carbon atoms, is reacted with an aromatic amine having the formula $R_1NH_2$, and the unreacted dialkyl carbonate and the concurrently produced alcohol are distilled off as the reaction proceeds, whereas in the second stage the residual products of the first stage are reacted with an aliphatic amine having the formula $R_2R_3NH$, characterized in that in the first stage the molar ratio of the dialkyl carbonate to the aromatic amine is comprised between 1:1 and 5:1, and from 50% to 95% of the aromatic amine concerned is converted, and in the second stage the as-produced intermediate carbamate coming from the first stage is reacted with the aliphatic amine in the presence of the residual unreacted aromatic amine.

2. Process according to claim 1, wherein the aromatic amine is a member selected from among aniline, 3,4 - bichloroaniline, 3 - chloro - 4 - methyl aniline, 3 - chloroaniline, 4 - chloroaniline and 2 - trifluoromethyl aniline.

3. Process according to claim 1, wherein the aliphatic amine is a member selected from among dimethylamine, diethylamine, di-nor.propylamine, di-nor.butylamine, nor-butylmethylamine, cyclohexylamine, methylamine, ethylamine, nor-propylamine and nor-butylamine.

4. Process according to claim 1, wherein the catalyst is a member selected from among sodium methoxide, sodium ethoxide, lithium methoxide, lithium ethoxide, zinc methoxide, zinc ethoxide, zinc acetate, zinc chloride and tin bichloride.

5. Process according to claim 1, wherein for both stages a quantity of catalyst is used, ranging from 1 to 10 mols per 100 mols of the aromatic amine.

6. Process according to claim 1, wherein the first stage is carried out at a temperature from 120 to 160°C with from 5 to 10 mols of catalyst per 100 mols of aromatic amine if an alkoxide, oxide or hydroxide catalyst is adopted, or with from 1 to 5 mols of Lewis' acid catalyst per 100 mols of aromatic amine, for a time from 1 to 6 hours.

7. Process according to claim 1, wherein the second stage is carried out at a temperature from 120°C to 140°C and for a time from 1 to 3 hours.

**Patentansprüche**

1. Katalytisches Verfahren zur Herstellung von asymmetrisch substituierten Harnstoffen mit der allgemeinen Formel:

$$HR_1NCONR_2R_3,$$

worin $R_1$ einen gegebenenfalls in seinem Ring substituierten Arylrest bedeutet;

$R_2$ einen Alkylrest mit 1 bis 10 Kohlenstoffatomen oder einen Cycloalkylrest mit 5 oder 6 Kohlenstoffatomen bedeutet, und

$R_3$ ein Wasserstoffatom oder $R_2$ bedeutet, nach einem Zweistufenverfahren, wobei in der

ersten Stufe ein Dialkylcarbonat miz der Formel RO—CO—OR, worin R einen Alkylrest mit 1 bis 4 Kohlenstoffatomen bedeutet, mit einem aromatischen Amin der Formel $R_1NH_2$ umgesetzt und nicht umgesetztes Dialkylcarbonat und gleichzeitig gebildeter Alkohol mit fortschreitender Reaktion abdestilliert werden, wogegen in der zweiten Stufe die aus der ersten Stufe verbleibenden Produkte mit einem aliphatischen Amin der Formel $R_2R_3NH$ umgesetzt werden, dadurch gekennzeichnet, daß in der ersten Stufe das Molverhältnis des Dialkylcarbonats zu aromatischem Amin zwischen 1:1 und 5:1 beträgt und daß von 50 bis 95% des jeweiligen aromatischen Amins umgewandelt werden, und daß in der zweiten Stufe das aus der ersten Stufe kommende Carbamat - Zwischenprodukt mit dem aliphatischen Amin in Anwesenheit des restlichen, nicht umgesetzten aromatischen Amins umgesetzt wird.

2. Verfahren nach Anspruch 1, worin das aromatische Amin ein unter Anilin, 3,4 - Dichloranilin, 3 - Chloro - 4 - methylanilin, 3 - Chloranilin, 4 - Chloranilin und Trifluoromethylanilin ausgewähltes Glied ist.

3. Verfahren nach Anspruch 1, worin das aliphatische Amin ein unter Dimethylamin, Diethylamin, Di-n-propylamin, Di-n-butylamin, n-Butyl-methylamin, Cyclohexylamin, Methylamin, Ethylamin, Propylamin und Butylamin ausgewähltes Glied ist.

4. Verfahren nach Anspruch 1, worin der Katalysator ein unter Natriummethoxide, Natriumethoxid, Lithiummethoxid, Lithiumethoxid, Zinkmethoxid, Zinkethoxid, Zinkacetat, Zinkchlorid und Zinnchlorid ausgewähltes Glied ist.

5. Verfahren nach Anspurch 1, worin für beide Verfahrensstufen eine Katalysatormenge verwendet wird, die von 1 bis 10 Mol je 100 Mol des aromatischen Amins beträgt.

6. Verfahren nach Anspruch 1, worin die erste Stufe bei einer Temperatur von 120 bis 160°C mit von 5 bis 10 Mol Katalysator je 100 Mol aromatisches Amin ausgeführt wird, wenn ein Alkoxid-, Oxid- oder Hyroxidkatalysator angewendet wird, oder mit von 1 bis 5 Mol eines Lewis - Säure - Katalysators je 100 Mol aromatisches Amin während 1 bis 6 Stunden ausgeführt wird.

7. Verfahren nach Anspruch 1, worin die zweite Stufe bei einer Temperatur von 120 bis 140°C während einer Zeit von 1 bis 3 Stunden ausgeführt wird.

**Revendications**

1. Procédé catalytique pour la préparation d'urées asymétriques substituées de formule générale

$$HR_1NCONR_2R_3$$

dans laquelle $R_1$ est un radical aryle éventuellement substitué sur son noyanu;

$R_2$ est un radical alkyle ayant de 1 à 10 atomes de carbone,

ou un radical cycloalkyle ayant 5 ou 6 atomes de carbone; et

$R_3$ est un atome d'hydrogène ou $R_2$;

par une opération en deux étapes, dans la première étape de laquelle on fait régagir un carbonate de dialkyle de formule RO—CO—OR, R étant un radical alkyl ayant de 1 à 4 atomes de carbone, avec une amine aromatique de formule $R_1NH_2$, et on élimine par distillation, à mesure que la réaction se déroule, le carbonate de dialkyle qui n'a par réagi et l'alcool produit simultanément, tandis que dans la deuxième étape, on fait réagir les produits restants de la première étape avec une amine aliphatique de formule $R_2R_3NH$, caractérisé par le fait que dans la première étape la raport molaire du carbonate de dialkyle à l'amine aromatique est compris entre 1:1 et 5:1, et que 50 à 95% de l'amine aromatique concernée sont transformés et dans la deuxième étape, on fait réagir tel quel le carbamate intermédiaire provenant de la première étape avec l'amine aliphatique, en présence de l'amine aromatique résiduelle qui n'a pas réagi.

2. Procédé selon la revendication 1, dans lequel l'amine aromatique est un composé choisi parmi l'aniline, la 3,4 - dichloraniline, la 3 - chloro - 4 - méthylaniline, la 3 - chloraniline, la 4 - chloraniline et la 2 - trifluorométhylaniline.

3. Procédé selon la revendicatin 1, dans lequel l'amine aliphatique est un composé choisi parmi la diméthylamine, la diéthylamine, la di-n-propylamine, la di-n-butylamine, la n - butyl - méthylamine, la cyclohexylamine, la méthylmaine, l'éthylamine, le n - propylamine et le n - butylamine.

4. Procédé selon la revendication 1, dans lequel le catalyseur est un composé choisi parmi le méthoxyde de sodium, l'éthoxyde de sodium, le méthoxyde de lithium, l'éthoxyde de lithium, le méthoxyde de zinc, l'éthoxyde de zinc, l'acétate de zinc, le chlorure de zinc et le chlorure stanneux.

5. Procédé selon la revendication 1, dans lequel on utilise pour les deux étapes une quantité de catalyseur allant de 1 à 10 moles pour 100 moles de l'amine aromatique.

6. Procédé selon la revendication 1, dans lequel la première étape est effectuée à une température de 120 à 160°C avec 5 à 10 moles de catalyseur pour 100 moles d'amine aromatique si l'on choisit un catalyseur de type alcoxyde, oxyde ou hydroxyde, ou avec 1 à 5 moles de catalyseur de type acide de Lewis, pour 100 moles d'amine aromatique, pendant une durée allant de 1 à 6 heures.

7. Procédé selon la revendication 1, dans lequel on effectue la deuxiéme étape à une température allant de 120 à 140°C et pendant une durée allant de 1 à 3 heures.